# EUROPEAN PATENT APPLICATION

(11) **EP 2 803 365 A1**
(43) Date of publication of application: **19.11.2014**
(21) Application number: 13305607.7
(22) Date of filing: 14.05.2013
(51) Int. Cl.: A61K 45/06, A61K 31/546, A61K 31/5513, A61P 31/00

(54) **Use of clonazepam in combination with antibiotic in the treatment of bacterially induced meningitis**

(71) Applicant: COMMISSARIAT A L'ENERGIE ATOMIQUE ET AUX ENERGIES ALTERNATIVES, 75015 Paris (FR)
(72) Inventor: Mouthon, Franck, 92100 BOULOGNE-BILLANCOURT (FR); Charveriat, Matthieu, 92130 ISSY LES MOULINEAUX (FR)
(74) Representative: Regimbeau

(57) **Abstract**

The present invention relates to the use of a well-known benzodiazepine compound, namely clonazepam, in combination with antibiotic agents, for treating bacterially induced meningitis.

## Description

### Prior Art Description

Meningitis is the inflammation of the protective membranes covering the central nervous system, known collectively as the meninges. Meningitis may develop in response to a number of causes, most prominently bacteria and viruses. This is a potentially serious condition owing to the proximity of the inflammation to the brain and spinal cord.

Bacterial meningitis is a serious threat to global health accounting for an estimated 171,000 deaths worldwide per year. The potential for serious neurological damage or even death necessitates prompt medical attention and evaluation. Bacterial meningitis is typically treated with antibiotics (such as ceftriaxone and cefotaxime) and requires close observation.

Numerous microorganisms may cause bacterial meningitis, but *Streptococcus pneumoniae* ("pneumococcus") and *Neisseria meningitidis* ("meningococcus") are the most common pathogens in patients without immune deficiency. *Streptococcus agalactiae, Haemophilus influenza* of type B and *Listeria monocytogenes* can also be involved.

*Streptococcus pneumoniae* is the prevalent organism responsible for invasive bacterial infection in young children. Mortality in children with pneumococcal meningitis is at least twice as high as in meningococcal meningitis and the survivors have the highest incidence of sequelae. Penicillin-resistant pneumococci are often multi-resistant, therefore posing serious problems for therapy. Resistance to macrolides is also widespread, being particularly high in the Mediterranean region.

Vaccines to protect against pneumococcus or meningococcus infections have been available for many years. Yet, prophylactic vaccination is often expensive and therefore not widespread enough, especially in developing countries.

Efficient curative treatments are therefore still needed to get rid of this lethal disease once it is diagnosed.

Curative antibiotics have been approved in Europe and in the United States, and many other are under development (for example, meropenem, ertapenem, moxifloxacine, Gemifloxacine, Garenoxacine, Daptomycine, Oritavancine, PTK 796, and GSK-1322322).

Antibiotic-based treatments are however not as efficient as they should be to definitively eradicate bacterial meningitis, so that this disease is still a worldwide threat for the human health, notably for young children and inhabitants of developing countries: the yearly incidence of bacterial meningitis in 2010 was estimated to be 2.6-6.0 cases per 100 000 in Europe and ten times higher in less developed countries.

To improve antibiotic-based treatments, several adjuvant compounds have been proposed.

It has been shown, for example, that prostane derivatives such as iloprost can be used as adjuvant treatment of bacterially induced meningitis (EP 0 852 497). Also, monoclonal antibodies anti-TNF can serve as efficient agent in established bacterial meningitis infections, when administered at least five hours after the antibiotics (EP 0 585 705).

Adjuvant treatment with corticosteroids (usually dexamethasone) was thought to reduce rates of mortality, severe hearing loss and neurological damage in adolescents and adults from high income countries which have low rates of HIV (the likely mechanism being the suppression of overactive inflammation). Yet, their use in children from low-income countries was not supported by evidence. Even in high-income countries, the benefit of corticosteroids was only seen when they were given prior to the first dose of antibiotics. Other uses are controversial. Van de Beek et al. have recently suggested that its use - in combination with antibiotics - does not seem to significantly reduce death or neurological disability for patients suffering from acute bacterial meningitis (*Lancet Neurol.* 2010). Yet dexamethasone was the only adjuvant to antibiotic treatments having been approved so far.

Finding more efficient and cheaper treatments for curing bacterial meningitis was therefore an urgent need. In this context, the present inventors have shown that low doses of clonazepam, a well-known and easily available anti-epileptic molecule, greatly enhance the efficiency of meningitis treatments in an *in vivo* system. Surprisingly, this curative effect is observed at a dose where no anti-epileptic effect is observed, suggesting that both mechanisms are not related.

### Summary of the invention

In a first aspect, the present invention concerns a combination product containing clonazepam and at least one antibiotic agent, for its use as a medicament, wherein clonazepam is present in an amount which does not induce anti-epileptic activity.

In a preferred embodiment, this combination product is used for treating patients suffering from bacterially induced meningitis.

In another preferred embodiment, clonazepam and said at least antibiotic agent are administered separately. Preferably, clonazepam is administered intravenously.

In a second aspect, the present invention is drawn to a pharmaceutical product containing clonazepam and at least one antibiotic agent, as combination products for simultaneous, separate or sequential use over time for treating patients suffering from bacterially induced meningitis, wherein clonazepam is present in an amount which does not induce anti-epileptic activity.

In preferred embodiments, the combination or the pharmaceutical product(s) of the invention contain(s) at least one antibiotic agent chosen among: cephalosporin, aminoglycosides, carbapenem, aminopenicillin glycopeptides, and nitroimidazoles. Preferably, said antibiotic agent is ceftriaxone.

In a third aspect, the present invention is drawn to clonazepam, for its use:
- as adjuvant to at least one antibiotic agent, or
- to improve the survival of patient with bacterial meningitis treated with one antibiotic or a combination of at least two antibiotics.

In this case, clonazepam is present in an amount which does not induce anti-epileptic activity.

### Figure legends

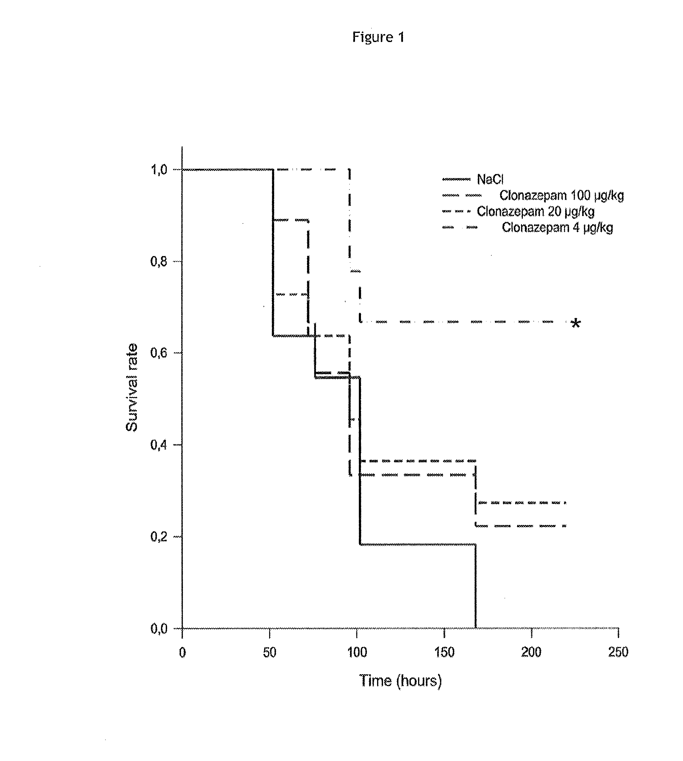

**Figure 1** discloses the Kaplan-Meier survival rate of Swiss mice inoculated with *Streptococcus Pneumoniae* bacteria. The antibiotic ceftriaxone 4 mg/day, 0.4 mg/day and 0.4mg/day was respectively administered intraperitoneally 24, 48 and 72 hours later ; either NaCl or clonazepam at different doses (4, 20 and 100 µg/kg) were administered intraperitoneously 25 hours after inoculation (*:p=0,04, Logrank test).

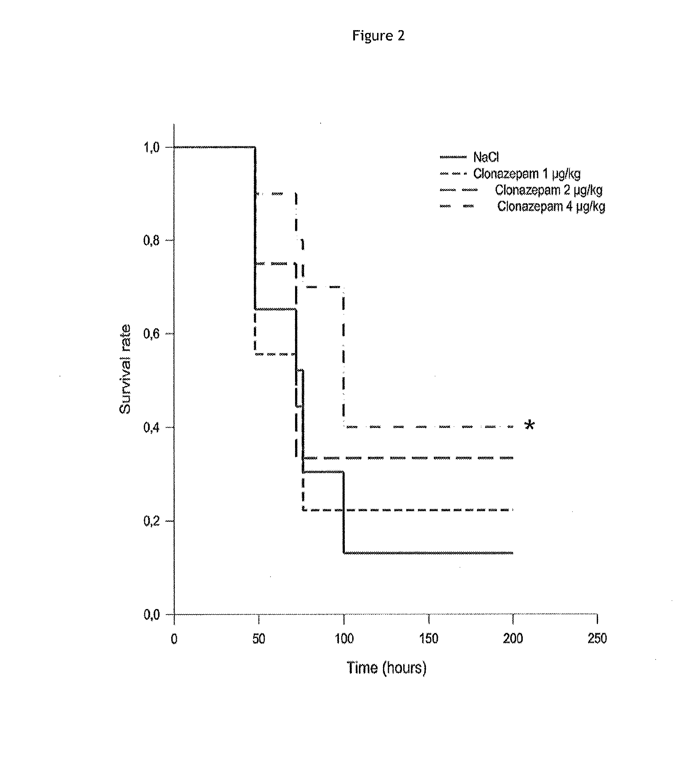

**Figure 2** discloses the Kaplan-Meier survival rate of Swiss mice inoculated with *Streptococcus Pneumoniae* bacteria. The antibiotic ceftriaxone 4 mg/day, 0.4 mg/day and 0.4mg/day was respectively administered intraperitoneally 24, 48 and 72 hours later ; either NaCl or clonazepam at different doses (1, 2 and 4 µg/kg) were administered intraperitoneously 25 hours after inoculation (*:p=0,045, Logrank test).

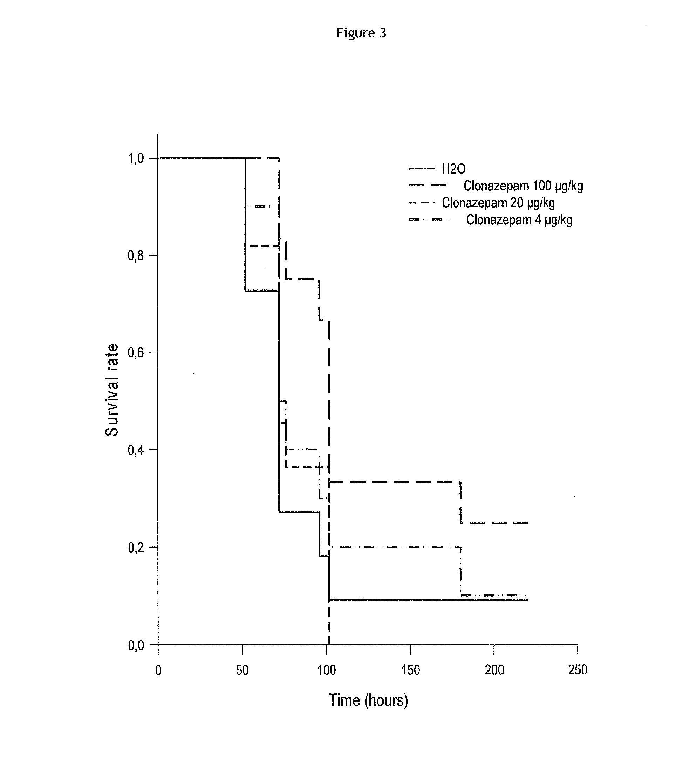

**Figure 3** discloses the Kaplan-Meier survival rate of Swiss mice inoculated with *Streptococcus Pneumoniae* bacteria. The antibiotic ceftriaxone 4 mg/day, 0.4 mg/day and 0.4mg/day was respectively administered intraperitoneally 24, 48 and 72 hours later ; either H₂O or clonazepam at different doses (4, 20 and 100 µg/kg) were administered orally 25 hours after inoculation (*:p=0,04, Logrank test).

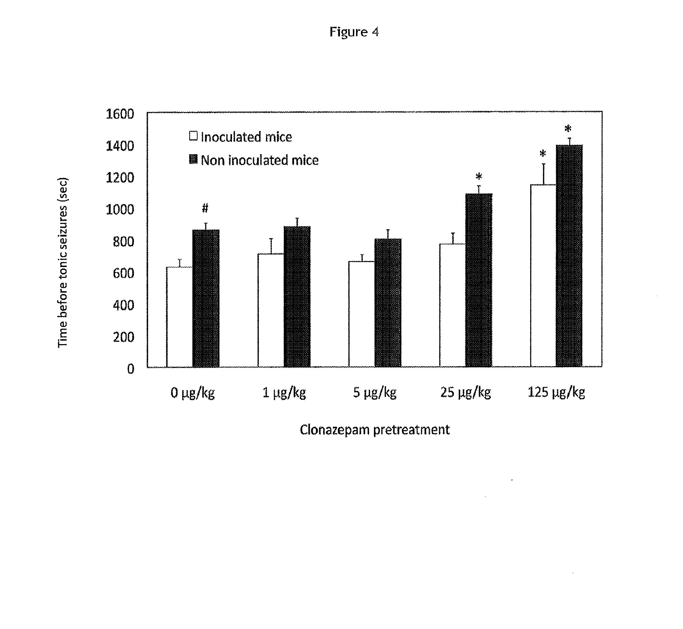

**Figure 4** discloses the time lapsed before tonic seizures for *Streptococcus Pneumoniae* inoculated mice ("inoculated") or non inoculated mice having received or not a pretreatment with increasing doses of clonazepam (1, 5, 25, 125 µg/kg) 25 hours after inoculation and being thereafter treated with increasing doses of pentylenetetrazol (PTZ) each three minutes. (*:p<0,05, Student test, between non inoculated mice 0 µg/kg and x µg/kg, and between inoculated mice 0 µg/kg and x µg/kg; #: p<0,05 (between non inoculated mice and inoculated mice for the 0 µg/kg condition; Mann-Whitney test).

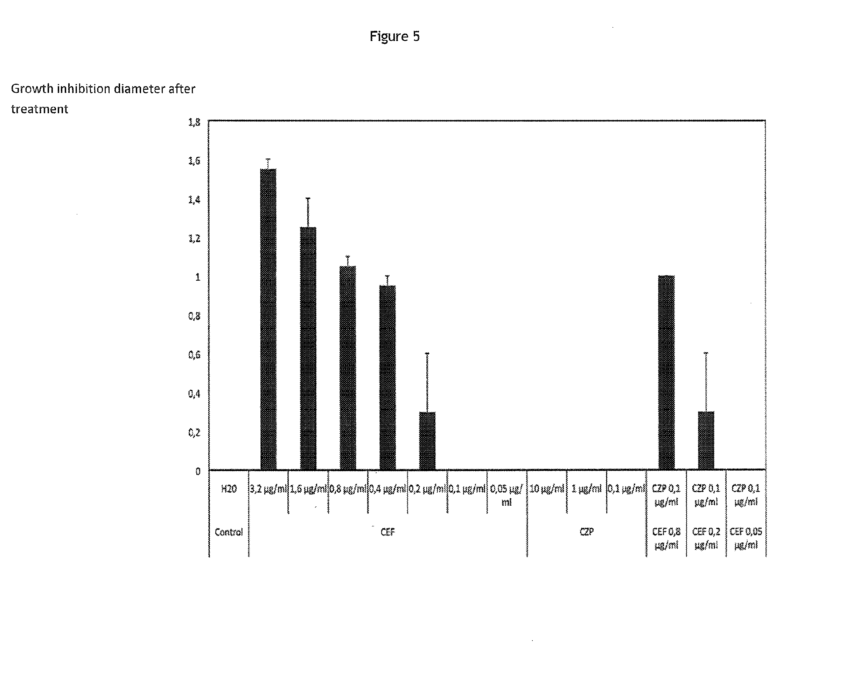

**Figure 5** discloses the growth inhibition diameter of *Streptococcus Pneumoniae* bacteria after treatment with ceftriaxone (CEF) and/or clonazepam (CZP) on blood-containing gelose.

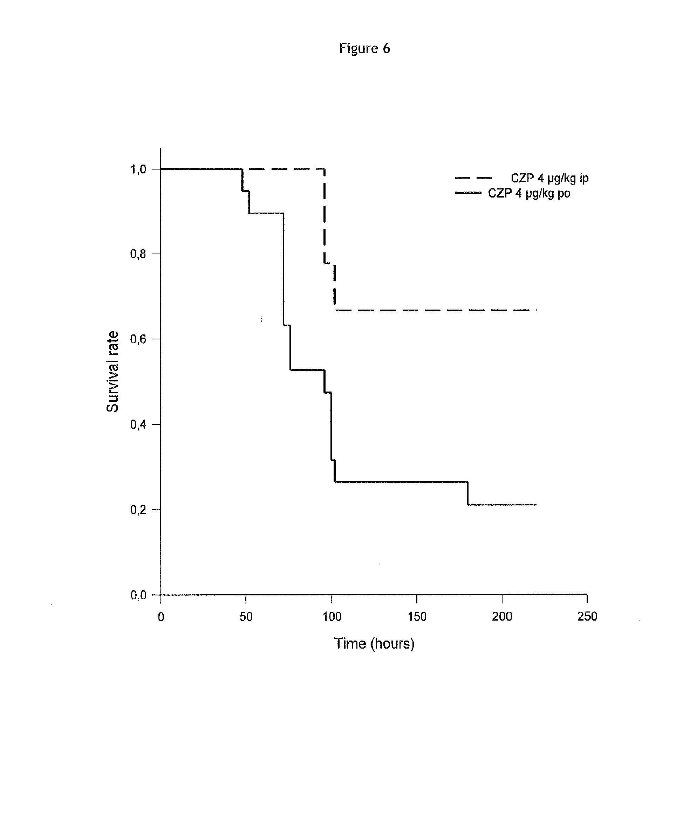

**Figure 6** discloses the Kaplan-Meier survival rate of Swiss mice inoculated with *Streptococcus Pneumoniae* bacteria and treated 24, 48 and 72 hours later with the antibiotic ceftriaxone 4 mg/day, 0.4 mg/day and 0.4mg/day respectively ; clonazepam (4 µg/kg) was administered intraperitoneously (ip) or orally (po) 25 hours after inoculation.

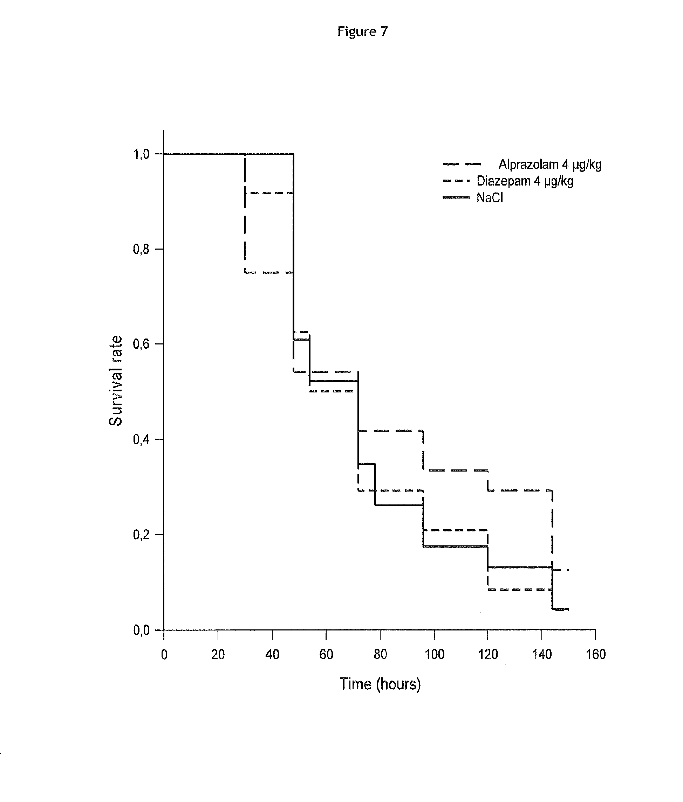

**Figure 7** discloses the Kaplan-Meier survival rate of Swiss mice inoculated with *Streptococcus Pneumoniae* bacteria, and treated 24, 48 and 72 hours later with the antibiotic ceftriaxone 4 mg/day, 0.4 mg/day and 0.4mg/day respectively; NaCl or alprazolam (4 µg/kg) or diazepam (4 µg/kg) was administrated intraperitoneously 25 hours after inoculation.

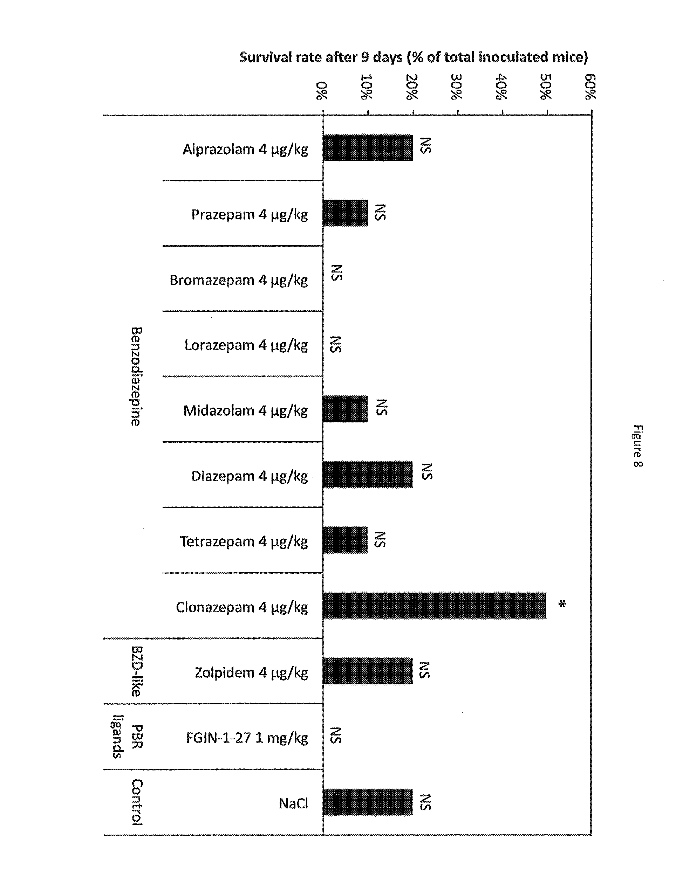

**Figure 8** discloses the survival rate of Swiss mice 9 days after being inoculated with *Streptococcus Pneumoniae* bacteria, and subsequently treated 24, 48 and 72 hours later with the antibiotic ceftriaxone 4 mg/day, 0.4 mg/day and 0.4mg/day respectively ; either NaCl or clonazepam (4 µg/kg) or alprazolam (4 µg/kg) or prazepam (4 µg/kg ), or bromazepam (4 µg/kg) or lorazepam (4 µg/kg ), or midazolam (4 µg/kg ) or diazepam (4 µg/kg) or tetrazepam (4 µg/kg) or the benzodiazepine-like zolpidem (4 µg/kg) or the PBR agonist ligand FGIN-I-27 (1 mg/kg) was administrated 25 hours after inoculation.

### Description of the invention

The present inventors have shown that low doses of clonazepam greatly potentiate antibiotic-based treatments of bacterial meningitis in the murine Swiss model.

Clonazepam is a benzodiazepine drug reducing neuro-inflammation, having in particular anxiolytic, anticonvulsant, muscle relaxant, and hypnotic properties. It is therefore often used as a second-line treatment of epilepsy. It is also used for the treatment of absence seizure, panic disorder, anxiety disorders, mania or acute psychosis (together with firstline drugs such as lithium, haloperidol or risperidone), or for the management of the visual effects of long term hospitalisation, e.g. hyperekplexia, parasomnia, restless legs syndrome, bruxism, rapid eye movement behavior disorder, acute and chronic akathisia induced by neuroleptics, and/or spasticity related to amyotrophic lateral sclerosis (see US 3,121,114 or, for example, Morishita S. et al, Human psychopharmacol. 2009, Dreifuss FE et al, Neurology 1975, Riss J. et al, Acta Neurol. Scand. 2008). Clonazepam is currently available as tablets and orally disintegrating tablets (wafers), oral solution (drops), as well as solution for injection or intravenous infusion.

Clonazepam was approved in the United States as a generic drug in 1997 and is now manufactured and marketed by several companies. It is marketed under the trade name Klonopin in the United States and Rivotril in Australia, Brazil, Canada and Europe (and in countries like Serbia, Macedonia, Croatia and Montenegro). Other names such as Ravotril, Rivatril, Clonex, Paxam, or Kriadex are known throughout the rest of the world. Clonazepam has an unusually long half-life of 18-50 hours, making it generally considered to be among the long-acting benzodiazepines. Clonazepam is a chlorinated derivative of nitrazepam and therefore a chloro-nitrobenzodiazepine of formula I:

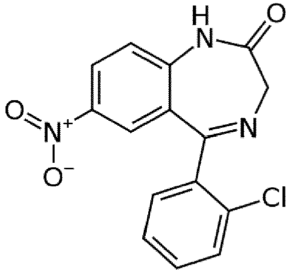

Clonazepam (5-(2-chlorphenyl)-1,3-dihydro-7-nitro-2H-1,4-benzodiazepine-2-one) is synthesized by following a standard scheme of making derivatives of 1,4-benzodiazepines, with the exception that the acceptor nitro group on C7 of the benzodiazepine system is introduced at the last stage of synthesis. The synthesis of clonazepam begins with 2-chloro-2'-nitrobenzophenone, which is reduced to 2-chloro-2'-aminobenzophenone by hydrogen over Raney nickel. The amino derivative is amidated by 2-bromoacetyl bromide to give the bromacetamide and is further converted into aminoacetamide upon reaction with ammonia. Upon reaction of this with pyridine, it is cycled into 5-(2-chlorophenyl)-2,3-dihydro-1H-1,4-benzodiazepine-2-one. The nitration of the resulting product in mild conditions (potassium nitrate in sulfuric acid) results in clonazepam. Its synthesis is more precisely disclosed for example in US 3,123,529.

Any pharmacologically acceptable salt of clonazepam is useful in the present invention. Although not limited to them, examples thereof include inorganic acid salts (such as hydrochlorides or nitrates), inorganic basic salts (such as sodium salts, potassium salts or calcium salts) and organic acid salts (such as acetates, benzoates, maleates, tartrates, fumarates or mesilates).

Like clonazepam, other benzodiazepine molecules have been shown to reduce neuroinflammation (e.g. midazolam and diazepam). This effect has been shown to be mediated by the peripheral benzodiazepin receptor PBR (also known as the "translocator protein" TSPO) (Torres S.R. et at, Eur. J. Pharmacol. 2000; Wilms H. et at, Neurobiol. Dis. 2003). Several PBR ligands have been described so far (e.g. PK11195, Ro5-4864, FGIN-1-27, DAA1106, and DPA-713, see for example James ML et al, Curr Med Chem 2006).

Yet, the present Inventors have shown that none of the other tested benzodiazepine molecules or PBR ligands is as efficient in potentiating the curative effect of antibiotics as clonazepam does. It can be concluded that this potentiation effect is not linked to the PBR / TSPO pathway.

Accordingly, the effect of clonazepam in combination with antibiotic treatment is thought to be highly specific of its particular chemical structure.

Moreover, and as shown in the experimental part below, this effect is not due to an enhancement of the antibacterial effect of the antibiotic. The clonazepam effect is therefore not directly acting on the infectious bacteria.

Finally, the present Inventors have shown that, surprisingly, the effect is observed only when clonazepam is used at a dose where no anti-epileptic effect can be obtained, suggesting that the potentiating effect is not related with its effects on epi lepsy.

Of note, the clonazepam effect is not linked with its anti-epileptic activity, with direct antibacterial activity, nor with direct PBR / TSPO activation.

In the context of the present invention, clonazepam (or its salt) is used in an amount "which does not induce any anti-epileptic activity". The suitable dose of clonazepam (or its salt) will of course depends on the host, the mode of administration and the type and severity of the condition that is to be treated. Generally, however, satisfactory results in animals are to be expected at daily doses of from 2 µg/kg to 25 µg/kg body weight, preferentially at 4 µg/kg body weight.

It is noteworthy that, in humans, the recommended clonazepam daily dose for inducing an anti-epileptic activity is reached when the plasmatic concentration thereof is comprised between 20 ng/mL and 70 ng/mL. *A contrario*, the clonazepam amount "which does not induce any anti-epileptic activity" will therefore generally correspond to a daily dose which, when assessed in the plasma of the patients, is strictly inferior to 20 ng/mL.

Clonazepam dose "which does not induce any anti-epileptic activity" will depend on the age of the patient. In the context of the invention, the term "children" matches to neonates to 15-year old children. The term "adult human" thus corresponds to people older than 15 years.

It is noteworthy that the recommended clonazepam daily dose for inducing an anti-epileptic activity in adult humans is 25 µg/kg at the beginning of the treatment (then 130-166 µg/kg). *A contrario*, the clonazepam amount "which does not induce any anti-epileptic activity" in adult humans will generally correspond to a daily dose which is strictly inferior to 25 µg/kg, preferably inferior to 20 µg/kg.

To show a significant potentiating effect, the amount of clonazepam should not be too low. Typically, in mice, satisfactory effects are obtained by using at least 2 µg/kg of clonazepam. It is thought that, by analogy, the amount of clonazepam in adult humans should be superior to 0.5 µg/kg, preferably superior to 1 µg/kg so that its potentiating effect would be significant.

Thus, in a more preferred embodiment, the clonazepam amount "which does not induce any anti-epileptic activity" in adult humans is comprised between 0.5 µg/kg and 25 µg/kg, preferably between 1 µg/kg and 20 µg/kg.

On another hand, it is noteworthy that the recommended clonazepam daily dose for inducing an anti-epileptic activity in children is 10 µg/kg at the beginning of the treatment (then 100-200 µg/kg). *A contrario*, the clonazepam amount "which does not induce any anti-epileptic activity" in children will generally correspond to a daily dose which is strictly inferior to 10 µg/kg, preferably inferior to 8 µg/kg.

It is thought that the clonazepam amount used in children should be superior to 0.2 µg/kg, preferably superior to 0.3 µg/kg so that its potentiating effect would be significant.

Thus, in a more preferred embodiment, the clonazepam amount "which does not induce any anti-epileptic activity" in children is comprised between 0.2 µg/kg and 10 µg/kg, preferably between 0.3 µg/kg and 8 µg/kg.

Clonazepam dose "which does not induce any anti-epileptic activity" will also depend on the way of administration, either orally or intravenously. Yet it is thought that the clonazepam amounts "which does not induce any anti-epileptic activity" will be comprised in about the same ranges than those described above. These doses are also transposable to the clonazepam salts.

The present Inventors have shown for the first time that clonazepam is helpful to improve the survival of a patient suffering from bacterial meningitis which is treated with antibiotic(s), and therefore, to potentiate the antibiotic treatment of bacterial meningitis.

Bacterial meningitis is a fulminant, often fatal pyogenic infection beginning in the meninges. A respiratory illness or sore throat often precedes the more characteristic symptoms of fever, headache, stiff neck, and vomiting. Adults may become desperately ill within 24 h, and children even sooner. Seizures occur in about 30% of the patients. Cranial nerve abnormalities and other focal deficits occur in 10 to 20% of the patients. In patients older than two years, changes in consciousness progress through irritability, confusion, drowsiness, stupor, and coma.

Many gram positive bacteria can cause meningitis, but the most common are Group B streptococci (during the first 2 months of life), *Neisseria meningitidis* (meningococci) and *Streptococcus pneumoniae* (pneumococci). Meningococci exist in the nasopharynx of about 5% of people and spread by respiratory droplets and close contact. Only a small fraction of carriers develop meningitis; what makes them susceptible is unknown. Meningococcal meningitis occurs most often during the first year of life. It also tends to occur in epidemics among closed populations (eg, in military barracks, college dormitories, and boarding schools). Pneumococci are the most common cause of meningitis in adults. Especially at risk are alcoholics and people with chronic otitis, sinusitis, mastoiditis, CSF leaks, recurrent meningitis, pneumococcal pneumonia, sickle cell disease, or asplenia. Incidence of pneumococcal meningitis is decreasing because of routine vaccination. Gram-negative organisms (most often *Escherichia coli, Klebsiella* sp., or *Enterobacter* sp.) can also cause meningitis in infants; in immunocompromised patients; or after CNS surgery, CNS trauma, bacteremia or hospital-acquired infections. *Pseudomonas* sp. occasionally causes meningitis in immunocompromised or colonized patients. *Haemophilus influenzae* type B meningitis, now uncommon because of widespread vaccination, can occur in immunocompromised patients or after head trauma in unvaccinated people. Staphylococci can cause meningitis after penetrating head wounds or neurosurgical procedures (often as part of a mixed infection) or after bacteremia (e.g., due to endocarditis). Listeria typically cause meningitis in the very young, the very old, and patients of any age who are immunocompromised because of chronic renal failure, hepatic disorders, or corticosteroid or cytotoxic therapy after organ transplantation.

Bacteria typically reach the meninges by hematogenous spread from sites of colonization in the nasopharynx or other foci of infection (e.g., pneumonia). Why some bacteria are more prone to colonize cerebrospinal fluid (CSF) is not clear, but binding pili and encapsulation appear to play a role. Bacteria can also enter CSF by direct extension from nearby infections (e.g., sinusitis, mastoiditis) or through exterior openings in normally closed CSF pathways (e.g., due to meningomyelocele, spinal dermal sinus, penetrating injuries, or neurosurgical procedures).

Meningitis treatment firstly requires antibiotics; corticosteroids are also commonly given.

The choice of empiric antibiotics depends on the suspected pathogen and patient age. Third-generation cephalosporins (e.g., ceftriaxone, cefotaxime) are effective against pathogens common in patients of all ages. Cefepime, a 4th-generation cephalosporin, can be substituted for a 3rd-generation cephalosporin in children and can be useful against *Pseudomonas* infection. However, because cephalosporin-resistant pneumococci are becoming increasingly prevalent, vancomycin (with or without rifampin) is usually added. Meropenem is also effective against *Pseudomonas* and many gram-negative bacteria. Ampicillin is added to get rid of *Listeria* sp. and aminoglycosides are still used empirically to cover gram-negative bacteria in neonates. When meningitis is due to a gram-negative anaerobe (e.g., because of otitis, sinusitis, or mastoiditis), meropenem is commonly added. For meningitis patients with a recent neurosurgical procedure or with an intraventricular shunt, vancomycin, meropenem, plus metronidazole provide coverage against staphylococci, gram-negative bacteria, and anaerobes.

Common antibiotic doses are listed in Table 1.

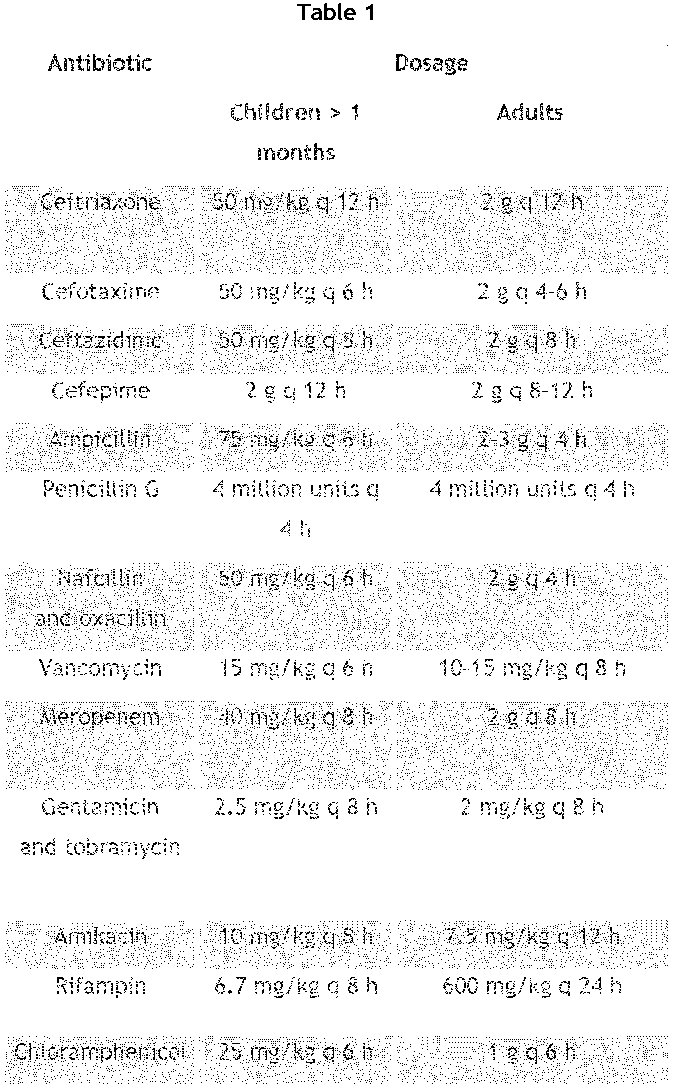

As mentioned previously, unprovoked seizures and epilepsy (recurrent unprovoked seizures) can be long-term sequelae of bacterial meningitis. The probability of developing an unprovoked seizure or epilepsy varies according to the etiologic agent responsible for meningitis, and this probability appears to be higher for *Streptococcus pneumonia* (Murthy J.M. Epilepsia 2008). In these cases, anti-epileptic treatments (e.g. benzodiazepine-based medicaments) can be proposed. However, in these cases, the amount of benzodiazepine (in particular clonazepam) is adjusted so to induce an anti-epileptic activity so that they are not encompassed in the present invention.

In a first aspect, the present invention concerns a therapeutic substance combination product containing clonazepam or a pharmaceutically salt thereof, and at least one antibiotic agent, for use as a medicament, wherein clonazepam or its salt is present in an amount which does not induce anti-epileptic activity. Preferably, said medicament is used for treating patients suffering from bacterially induced meningitis.

This combination product is for example a kit, containing, either in the same recipient or in two distinct recipients, clonazepam or a pharmaceutical salt thereof and at least one antibiotic.

In other words, the present invention covers the use of clonazepam or a pharmaceutically salt thereof and at least one antibiotic agent for the preparation of a medicament, wherein clonazepam or its salt is present in an amount which does not induce anti-epileptic activity. Preferably, said medicament is used for treating patients suffering from bacterially induced meningitis.

The present invention also discloses a method for treating a disease, said method comprising administering to a patient in need thereof clonazepam or a pharmaceutically salt thereof and at least one antibiotic agent, wherein clonazepam or its salt is present in an amount which does not induce anti-epileptic activity. Preferably, said disease is bacterially induced meningitis.

In a second aspect, the present invention discloses a pharmaceutical product containing clonazepam or a pharmaceutically salt thereof and at least one antibiotic agent, as combination products for simultaneous, separate or sequential use over time, for treating patients suffering from bacterially induced meningitis. In this aspect, clonazepam or its salt being present in a low amount, that is, in an amount which does not induce anti-epileptic activity (typically, lower than 25 µg/kg for an adult human, and lower than 10 µg/kg for a children).

In other words, the present invention targets the use of clonazepam or a pharmaceutically salt thereof and an antibiotic agent for the preparation of a medicament intended to be simultaneously, separately or sequentially used over time for treating patients suffering from bacterially induced meningitis, clonazepam or its salt being present in a low amount, that is, in an amount which does not induce anti-epileptic activity (typically, lower than 25 µg/kg for an adult human, and lower than 10 µg/kg for a children).

The present invention also discloses a method for treating patients suffering from bacterially induced meningitis, comprising the step of administering simultaneously, separately or sequentially over time a combination product containing at least clonazepam or a pharmaceutically salt thereof and an antibiotic agent to said patients, clonazepam or its salt being present in an amount which does not induce anti-epileptic activity (typically, lower than 25 µg/kg for an adult human, and lower than 10 µg/kg for a children).

In the case of simultaneous use, the different components of the treatment (namely the clonazepam or its salt and the antibiotic or a combination of at least two antibiotics) are administered to the patient simultaneously. According to this embodiment of the present invention, the components can be packaged together, in the form of a mixture, or separately, then mixed extemporaneously before being administered together to the patient. More commonly, the components are administered simultaneously, but separately. In particular, the routes of administration of the components may be different. The administration can also be performed at different sites. In another embodiment, the two active principles are administered sequentially or spaced apart over time, for example in the same day or at an interval ranging from several hours to several weeks, or even several months.

In a preferred embodiment, clonazepam is administered orally, enterally, parenterally or intravenously. Intranasal administration is not preferred. In a more preferred embodiment, clonazepam is administered either orally, or intravenously. Administration means of antibiotic(s) are well-known in the art and can be performed as described in the prior art. There is indeed no need to administer clonazepam and antibiotic(s) at the same time or in the same way (e.g. orally or intravenously). Suppositories, tablets, capsules, drops, injection solutions and oral suspensions are appropriate forms for administration of antibiotics.

By "intravenously", it is herein meant that the administration of the combination product of the invention is performed directly into the blood circulatory system as done, for example, via venous or arterial routes.

The combination product and the pharmaceutical product of the invention can also contain any useful physiologically tolerable pharmaceutical excipient and carriers.

As used herein, "physiologically tolerable pharmaceutical excipient" includes any and all solvents, buffers, salt solutions, dispersion media, coatings, antibacterial and antifungal agents, isotonic and absorption delaying agents, and the like that are physiologically compatible. The type of carrier can be selected based upon the intended route of administration. In various embodiments, the excipient is suitable for intravenous, intraperitoneal, subcutaneous, intramuscular, topical, transdermal or oral administration. Physiologically tolerable pharmaceutical excipients include sterile aqueous solutions or dispersions and sterile powders for the extemporaneous preparation of sterile injectable solutions or dispersion. Actual methods for preparing parenterally administrable compounds will be known or apparent to those skilled in the art and are described in more detail in for example, Remington's Pharmaceutical Science, 17th ed., Mack Publishing Company, Easton, Pa. (1985), and the 18th and 19th editions thereof, which are incorporated herein by reference.

Clonazepam (or its salt) and the antibiotic(s) in the composition product of the invention are preferably formulated in an effective amount. An "effective amount" refers to an amount effective, at dosages and for periods of time necessary, to achieve the desired result. For clonazepam, such "effective amount" refers to the amount which potentiates the antibiotic, without inducing any anti-epileptic activity, and without activating any PBR / TSPO pathway. Such amounts have been described above. For antibiotic, such "effective amount" refers to commonly used therapeutically effective amounts known in the art, for example those listed in table 1 above.

A "therapeutically effective amount" means an amount sufficient to influence the therapeutic course of a particular disease state, here bacterially induced meningitis. A therapeutically effective amount is also one in which any toxic or detrimental effects of the agent(s) are outweighed by the therapeutically beneficial effects.

In a third aspect, the present invention concerns the clonazepam compound as described above (5-(2-chlorphenyt)-1,3-dihydro-7-nitro-2H-1,4-benzodiazepine-2-one), or a pharmaceutically salt thereof, for use as adjuvant to at least one antibiotic agent, clonazepam being present in an amount which does not induce anti-epileptic activity.

In the context of the invention, an "adjuvant" treatment (or "adjunctive" treatment) corresponds to an "additional" treatment, antibiotic(s) being always present as baseline treatment against bacterially induced meningitis.

In this aspect, the present invention therefore concerns the use of the clonazepam molecule, or a pharmaceutically salt thereof, for the preparation of an adjuvant treatment to antibiotic agents.

A method for treating a patient suffering from a bacterial infection, comprising using clonazepam or a pharmaceutically salt thereof, in an amount which does not induce anti-epileptic activity, as adjuvant treatment to antibiotic agent, is also disclosed.

All bacterially-induced disease can be targeted by this adjuvant treatment, for example sepsis, pneumonia, anthrax, brucellosis, enteritis, botulism, colitis, gangrene, tetanus, diphtheria, nosocomial infections, meningitis, diarrhea, tularemia, upper respiratory tract infections, bronchitis, leptospirosis, listeriosis, legionnaire's disease, pontiac fever, tuberculosis, septic arthritis, salmonellosis, shigellosis, septicemia, endocarditis, impetigo, cystitis, otitis, sinusitis, pharyngitis, fever, syphilis, cholera, and plague, provided that they are of bacterial origin.

Preferably, said adjuvant compound is used in the treatment of bacterially induced meningitis.

Antibiotics, their action and uses are well-known many decades. Table 1 discloses examples of antibiotics and suitable doses that are commonly used for treating meningitis (e.g., from 10 to 50 mg/kg depending on the nature of the antibiotic). Each of the antibiotics described therein can be used in combination with clonazepam in the conditions of the invention.

In a preferred embodiment of the invention, clonazepam or its salt is combined with a beta-lactam antibiotic or a combination of at least two beta-lactam antibiotics.

In a more preferred embodiment, clonazepam or its salt is combined with at least one antibiotic chosen among:
- cephalosporin, such as ceftriaxone, cefotaxime, and cefepime,
- aminoglycosides, such as amikacin, gentamicin, kanamycin, neomycin, netilmicine, paromomycin, streptomycin, and tobramycin,
- carbapenem, such as meropenem, imipenem, ertapenem,
- aminopenicillin, such as ampicillin and amoxicillin,
- glycopeptides, such as vancomycin, and
- nitroimidazoles, such as metronidazole and rifampin.

This exhaustive list is however not limiting and any other efficient antibiotic or combination of antibiotics can be combined with clonazepam in order to treat bacterially-induced meningitis.

Combination of at least two antibiotics chosen among the above-mentioned antibiotics can also be used in combination with clonazepam.

In an even more preferred embodiment, clonazepam or its salt is combined with an antibiotic or a combination of at least two antibiotics of the cephalosporin family.

In the most preferred embodiment, clonazepam or its salt is combined with ceftriaxone or a combination of ceftriaxone with at least one antibiotic of the cephalosporin family.

In a fourth aspect, the present invention concerns the clonazepam compound as described above (5-(2-chlorphenyl)-1,3-dihydro-7-nitro-2H-1,4-benzodiazepine-2-one) or a pharmaceutically salt thereof for use for improving the survival of a patient suffering from bacterial meningitis, said patient being treated with an antibiotic or a combination of at least two antibiotics, clonazepam or its salt being used in an amount which does not induce anti-epileptic activity.

In this aspect, the present invention covers the use of clonazepam or a pharmaceutically salt thereof in the preparation of a potentiator of antibiotic-based treatment, clonazepam or its salt being used in an amount which does not induce anti-epileptic activity. Preferably, said potentiator is used for treating bacterially-induced meningitis.

A method of using clonazepam or a pharmaceutically salt thereof as a potentiator of antibiotic-based treatment in patients in need thereof is also disclosed. Preferably, said patients suffer from bacterially induced meningitis.

Importantly, this potentiating effect is not mediated by a direct antibacterial activity, nor by the activation of the PBR / TSPO receptors, nor by an anti-epileptic activity.

All the above-mentioned bacterially-induced disease can be targeted by this adjuvant treatment. Yet, said compound is preferably used for treating bacterially-induced meningitis.

In the context of the invention, the term "potentiate" means significantly increasing the effects of the antibiotic(s) administered before, simultaneously or after clonazepam. In the context of the invention, "potentiation" is achieved for example when, by comparing the survival rate after 9 days of mice inoculated with 1 Mc Farland bacteria, that are treated with effective amounts of either antibiotic(s) alone, or of the same antibiotic(s) plus the clonazepam, the said survival rate is enhanced by at least 20 %, and preferably at least 30 %, when the mice are treated with the antibiotic(s) and clonazepam, as compared with the survival of the mice that are treated only with the antibiotic(s).

In particular, the combination of the antibiotic(s) with clonazepam or its salt makes it possible to enhance the efficiency of the antibiotic(s), even at high doses, or, if necessary, to reduce the doses of said antibiotic(s) and therefore to limit the adverse effects of said antibiotic(s), and/or to reduce the effects of failure and resistance.

### Examples

### 1. Material and methods

### 1.1. Bacteria preparation

200 µL of suspended 1 Mc Farland bacteria (*Streptococcus Pneumoniae*, CRBIP, Institut Pasteur) were seeded overnight on blood Columbia plates (BioRad). 24h later, bacteria were suspended in 5 mL phosphate buffer solution (PBS), and diluted to obtain 1 Mc Farland 600 nm optical density (3,2.10⁷ bacteria/mL for this strain).

### 1.2. Preclinical model

A mice model mimicking human bacterial meningitis (Gerber G., Acta Neuropathol. 2001) was implemented; Swiss male mice were used, with food and water *ab libitum.* Mice were anaesthetized using isoflurane.

The Swiss mice model has been chosen for the following reasons:
- It is possible to induce bacterial meningitis in a direct and reproducible way (Gerber G., Acta Neuropathol. 2001).
- The induced meningitis is similar to human meningitis (Gerber G., Acta Neuropathol. 2001). In particular, the inoculated mice present several different symptoms / responses as infected humans. The data obtained with this model can therefore be transposed to human.
- Efficiency of antibiotic agents for treating bacterial meningitis is currently studied in mice (Shapiro M.A. et al, J. Antimicrob. Chemother. 2000).

A 1/80 dilution of the prepared bacteria was then performed, so as to obtain 10⁴ bacteria in 25 µL. 25 µL bacteria suspension were injected in the left brain hemisphere. 24h, 48h and 72h later, mice were treated with 4 mg/day, 0.4 mg/day and 0.4mg/day respectively of ceftriaxone. Clonazepam (or saline buffer, or water) was administrated 25h post inoculation, by intraperitoneal (ip) or oral (po) route. Mortality was assessed several times per day, during 9 days.

### 1.3. Anti-epileptic effect of clonazepam at low dose

Mice were inoculated, and 24h later pentylenetetrazol (PTZ) was administrated, according to protocol adapted from (Kosobud A.E. and Crabbe J.C., Brain Res. 1990):
PTZ was diluted at the dose of 30 mg/kg for 100 µl in saline buffer, and PTZ and clonazepam were administrated following this protocol:
   - t₀₋₆₀ₘᵢₙ : intraperitoneal (ip) pretreatment with clonazepam (1, 5, 25, 125 µg/kg) or saline buffer (n=8 per treatment)
   - t₀ : ip administration of 60 mg/kg of PTZ
   - t₀₊₆ₘᵢₙ : ip administration of 30 mg/kg of PTZ (therefore 90 mg/kg)
   - t₀₊₉ₘᵢₙ : ip administration of 30 mg/kg of PTZ (therefore 120 mg/kg)
   - t₀₊₁₂ₘᵢₙ : ip administration of 30 mg/kg of PTZ (therefore 150 mg/kg)
   - ... until the first seizure inducing mice tetany.

Injections every 3 minutes were performed, until a seizure induces a general tetany of the mice. At this point, the time of the beginning of this crisis was noted (and the animal was euthanized).

### 1.4. In vitro study of antibiotic activity of clonazepam and ceftriaxone/clonazepam combination

Antibiograms have been performed on the same *Streptococcus Pneumoniae* strain, on a Mueller-Hinton gelose plate of 90mm diameter (Biorad) as follows 40 µL of the prepared bacteria were added to 5mL PBS and the mix is spread on the gelose plate. The remaining liquid mix is thrown and the plate was incubated at 37°C during 15 minutes. Antibiotic paper disks of 6mm diameter (Dominique Dutscher) were then overwhelmed with 25 µL of each molecule / combination and placed on the Mueller-Hinton gelose for 24 hours. The inhibition diameter of bacterial cell growth around the disks was then measured.
- Ceftriaxone (3,2 - 1,6 - 0,8 - 0,4 - 0,2 - 0,1 - 0,05 µg/mL)
- Clonazepam (10 - 1 - 0,1 µg/mL)
- Ceftriaxone (0,8 - 0,2 - 0,05 µg/mL) + Clonazepam (0,1 µg/mL)

### 1.5. Statistical analyses

Statistical analyses were implemented using Sigma Plot software.

### 2. Results

### 2.1. Use of clonazepam as adjuvant to antibiotherapy for treating bacterial meningitis

Figure 1 shows the survival of Swiss mice inoculated with *Streptococcus Pneumoniae* bacteria, in which ceftriaxone was administered in combination with either NaCl or clonazepam at different doses (4, 20 and 100 µg/kg).

It has been observed that survival increase for the mice receiving the combination [ceftriaxone + clonazepam] 4 µg/kg, this increase being reproducible and significant (*:p=0,04, Logrank test). It is to be noted that clonazepam was not as efficient when administered in a superior amount (for example at 20 and 100 µg/kg).

On another hand, Figure 2 shows the survival of Swiss mice inoculated with *Streptococcus Pneumoniae* bacteria, in which ceftriaxone was administered in combination with low doses of clonazepam (1, 2 and 4 µg/kg). This data indicate that clonazepam is significantly less efficient when administered in an amount inferior to 4 µg/kg, for example at 1 or 2 µg/kg (*:p=0,045, Logrank test).

### 2.2. Influence of the way of administration

Two different administration ways were compared (oral vs intraperitoneal).

As shown on figure 3, the survival rate of inoculated Swiss mice orally treated with ceftriaxone in combination clonazepam (4 µg/kg) is lower than the one obtained for mice treated with intraperitoneally-administered combination (compare figure 1 and 3).

This is further disclosed on figure 6 where both curves corresponding to the same combination [clonazepam 4 µg/kg and ceftriaxone] are shown.

Consequently, intraperitoneal administration is preferred in mice.

### 2.3. Anti-epileptic effect of clonazepam in healthy or inoculated Swiss mice

As shown on Figure 4 for healthy mice (black bars), clonazepam has an anti-epileptic effect when administered in an amount which is at least of 25 µg/kg. A contrario, no effect was found for lower amounts (namely 5 and 1 µg/kg). This result confirms what was known in the literature (see for example Georgiev V.P. et al, Neuropeptides 1991).

When performed on inoculated mice, it was noticed that the epileptic threshold was lower than in healthy mice. Importantly, however, no epileptic effect is observed for clonazepam doses inferior to 5 µg/kg.

It can be concluded from these experiments that clonazepam, at the efficient dose of 4 µg/kg, has no epileptic effect.

### 2.4. Antibiotic activity of clonazepam in vitro

In order to better understand the functional mechanism responsible for the potentiation of the antibiotic agent, it was then assessed if clonazepam could have intrinsic antibiotic effects on its own.

Figure 5 discloses the growth inhibition diameter of *Streptococcus Pneumoniae* bacteria after treatment with ceftriaxone (CEF) alone, clonazepam (CZP) alone, or both.

As expected, ceftriaxone inhibit bacterial growth in a dose-dependent manner.

However, clonazepam has no intrinsic antibiotic activity for all the assessed doses. Nor is it able to potentiate the antibiotic activity of ceftriaxone in vitro (last three conditions).

### 2.5. Specificity of the clonazepam effect

As disclosed above, clonazepam is a high potency benzodiazepine. The combination of ceftriaxone with other benzodiazepine was tested to determine if the observed antibiotic potentiating effect was specific to clonazepam.

The combination of the antibiotic ceftriaxone with several known benzodiazepine molecules, namely alprazolam, prazepam, bromazepam, lorazepam, midazolam, diazepam, or tetrazepam, was therefore tested for treating Swiss mice inoculated with *Streptococcus Pneumoniae* bacteria.

Also, one benzodiazepine-like agent, zolpidem, was tested (in combination with ceftriaxone).

Finally, the PBR agonist ligand FGIN-I-27 (James M.L. et al, Curr. Med. Chem. 2006; Torres S.R. et al, Eur. J. Pharmacol. 2000) was also tested (in combination with ceftriaxone).

Importantly, the results shown on figures 7 and 8 demonstrate that none of the tested benzodiazepine or benzodiazepine-like molecules nor the PBR agonist ligand were efficient in potentiating the antibiotic effect and enhancing the survival of the inoculated mice.

This clearly shows that the effect of clonazepam is specific and might not be related to its benzodiazepine structure.

### 3. Conclusions

All the above-mentioned experiments lead to the following conclusions:
- Clonazepam is an effective adjuvant to antibiotic treatments used in bacterial meningitis, at a dose of 4 µg/kg in mice,
- Clonazepam does not potentiate the in vitro antibiotic activity of ceftriaxone, and does not show any antibiotic activity on its own.
- The efficiency of clonazepam as adjuvant is independent of its epileptic activity.

### BIBLIOGRAPHIC REFERENCES

Van de Beek et al. Adjunctive dexamethasone in bacterial meningitis: a meta-analysis of individual patient data. Lancet Neurol. 2010 Mar;9(3):254-63.

Morishita S. et al. Clonazepam as a therapeutic adjunct to improve the management of depression: a brief review. Human psychopharmacol. 2009 Apr;24(3):191-8.

Dreifuss FE et al, Serum clonazepam concentrations in children with absence seizures. Neurology 1975 Mar;25(3):255-8.

Riss J. et al, Benzodiazepines in epilepsy: pharmacology and pharmacokinetics. Acta Neurol. Scand. 2008 Aug;118(2):69-86.

Torres S.R. et al, Anti-inflammatory effects of peripheral benzodiazepine receptor ligands in two mouse models of inflammation.Eur. J. Pharmacol. 2000 Nov 17;408(2):199-211.

Wilms H. et al, Involvement of benzodiazepine receptors in neuroinflammatory and neurodegenerative diseases: evidence from activated microglial cells in vitro. Neurobiol. Dis. 2003 Dec;14(3):417-24.

James ML et al, Development of ligands for the peripheral benzodiazepine receptor. Curr Med Chem 2006 ; 13(17):1991-2001.

Murthy J.M. Bacterial meningitis and epilepsy. Epilepsia 2008 Aug;49 Suppl 6:8-12.

Gerber G., A mouse model of Streptococcus pneunomiae meningitis mimicking several features of human disease. Acta Neuropathol. 2001; 101; 499-508

Shapiro M.A. et al, Comparative therapeutic efficacy of clinafloxacin in a pneumococcal meningitis mouse model. J. Antimicrob. Chemother. 2000 Apr;45(4):489-92.

Kosobud A.E. and Crabbe J.C., Genetic correlations among inbred strain sensitivities to convulsions induced by 9 convulsant drugs. Brain Res. 1990 Aug 27;526(1):8-16.

Georgiev V.P. et al, Interactions between angiotensin II, diazepam, clonazepam and di-n-propylacetate in pentylenetetrazol kindling seizures in mice. Neuropeptides 1991 Apr;18(4):187-91.

## Claims

1. A combination product containing clonazepam or a pharmaceutically salt thereof, and at least one antibiotic agent, for use as a medicament, wherein clonazepam or its salt is present in an amount which does not induce anti-epileptic activity.

2. The combination product according to claim 1, for use for treating patients suffering from bacterially induced meningitis.

3. The combination product for use according to claims 1 or 2, wherein clonazepam or its salt and said at least one antibiotic agent are administered separately.

4. The combination product for use according to any one of claim 1 to 3, wherein clonazepam or its salt is administered intravenously.

5. The combination product for use according to any one of claim 1 to 4, wherein said at least one antibiotic agent is chosen among: cephalosporin, aminoglycosides, carbapenem, aminopenicillin glycopeptides, and nitroimidazoles.

6. The combination product for use according to any one of claim 1 to 5, wherein said at least one antibiotic agent is ceftriaxone.

7. Pharmaceutical product containing clonazepam or a pharmaceutically salt thereof and at least one antibiotic agent, as combination products for simultaneous, separate or sequential use over time, as a medicament for treating patients suffering from bacterially induced meningitis, wherein clonazepam or its salt is present in an amount which does not induce anti-epileptic activity.

8. Pharmaceutical product according to claim 7, wherein said at least one antibiotic agent is chosen among: cephalosporin, aminoglycosides, carbapenem, aminopenicillin glycopeptides, and nitroimidazoles.

9. Pharmaceutical product according to claim 7 or 8, wherein said at least one antibiotic agent is ceftriaxone.

10. Pharmaceutical product according to any one of claim 7 to 9, wherein clonazepam or its salt is administered intravenously.

11. Clonazepam or a pharmaceutically salt thereof, for use as adjuvant to at least one antibiotic agent, clonazepam or its salt being present in an amount which does not induce anti-epileptic activity.

12. Clonazepam or a pharmaceutically salt thereof, for improving the survival of a patient suffering from bacterial meningitis, said patient being treated with an antibiotic or a combination of at least two antibiotics, clonazepam or its salt being present in an amount which does not induce anti-epileptic activity.

13. Clonazepam or a pharmaceutically salt thereof according to claims 11 or 12, for use in the treatment of bacterially induced meningitis.

14. Clonazepam or a pharmaceutically salt thereof for use according to any one of claims 11 to 13, wherein said at least one antibiotic agent is ceftriaxone.
